# EUROPEAN PATENT SPECIFICATION

(11) **EP 3 081 698 B1**
(45) Date of publication and mention of the grant of the patent: **13.06.2018**
(21) Application number: 16165407.4
(22) Date of filing: 14.04.2016
(51) Int. Cl.: G01N 3/42, G01N 33/24, E02D 1/02

(54) **SOIL PROBING DEVICE HAVING THREADED MALE AND FEMALE BAYONET COLUMNS**
BODENSONDIERUNGSVORRICHTUNG MIT GEWINDESTECKER- UND BUCHSENBAJONETTSÄULEN
DISPOSITIF DE SONDAGE DU SOL À COLONNES FILETÉES À BAÏONNETTE MÂLE ET FEMELLE

(30) Priority: 17.04.2015 NL 2014658
(43) Date of publication of application: 19.10.2016
(73) Proprietor: A.P. van den Berg Holding B.V., 8445 PK Heerenveen (NL)
(72) Inventor: HOEKSTRA, Arend Tjeerd, 9251 NG Burgum (NL)
(74) Representative: EP&C

(56) References cited:
- EP-A1- 0 989 240
- WO-A1-00/17481
- AU-A1- 2014 202 985
- US-A- 1 849 068

## Description

The present invention relates to the field of soil probing devices for determining geophysical and geotechnical properties of soil during penetration of a probing rod into the ground at land or at sea floors.

Such soil probing devices are known in various embodiments. For example EP 0 989 240, that was filed in the name of the present applicant, shows a soil probing device that has two sets of clamping members that can be moved up and down independently from each other and that are designed to alternately clamp a probing rod and push it gradually into the ground. Cone penetration measurements are performed during this penetration for determining the desired geophysical and geotechnical properties of the soil. The soil probing device can be supported directly onto a piece of land via a frame or can be mounted on a vehicle like a caterpillar-type. It can also be used for carrying out penetration measurements underwater, for example on a sea bed, provided that it is equipped with a special underwater drive unit. During penetration, the two sets of clamping members are controlled such that a first one of them gets moved down in a clamping position while taking the probing rod with it and pushing it into the ground, whereas a second one at the same time gets moved up along the probing rod in a release position. As soon as the first clamping set starts to reach its lowermost position, both clamping sets are controlled to switch functionality, that is to say that the second one then takes over the clamping of the probing rod and starts to push it further down into the ground, while the first one gets moved up along the probing rod in a release position. Thus the probing rod can be pushed in one continuous go into the ground. The probing rod here is formed by a measuring probe at its forward end and a plurality of distinctive straight rod sections of limited length that get connected thereto. With this, the rod sections are connected to one another by means of engageable male and female screw thread connections that are provided at free outer ends of the rods.

A disadvantage with this known device is that the screwing of the rod sections together is time-consuming. Also this screwing of the rod sections is difficult to automate, particularly when it is desired to perform the screwing of a new rod section to the probing rod at the same time that this probing rod is forced to penetrate at a constant speed into the ground in one continuous go. Another disadvantage is that during a removing of the probing rod out of the ground again, the unscrewing of the rod sections also is time-consuming and difficult to automate. Furthermore it then sometimes appears that the screw connections have gotten screwed together too tight and can only be loosened with great difficulty.

Another known soil probing device that has been successfully commercialized for several years in the market by applicant under the trademark "Roson", comprises a frame inside which two rotatable gripping wheels are provided as drive means. Those gripping wheels are positioned opposite one another. A probing rod is guided with a tight fit in between the gripping wheels. A rotating of the gripping wheels then forces the probing rod to penetrate in one continuous go into the ground. When to be used offshore for measurements on sea floors, it is known for this device to preassemble lengths of probing rod of up to 40 metres out of a plurality of rod sections inside an evenly long support mast that is placed upright on deck of or on-board of a ship. Subsequently the soil probing device together with the support mast and the preassembled length of probing rod inside it, gets lowered to the sea floor and there can start to perform a penetration test.

A disadvantage with this is that the preassembling of the probing rod on the ship is time-consuming and makes the operational costs for the ship expensive. Furthermore the preassembling process of the probing rod is sensitive for weather conditions and swells. Also the assembly of the preassembled probing rod and support mast is vulnerable because of its great length, not only during the preassembly process itself but also during its lowering towards the sea floor, which can be more than 2000 metres below. Further it is disadvantageous that a heavy winch is needed for lowering the assembly to the sea floor, while at the same time means need to be provided for keeping the support mast and the preassembled probing rod in an upright position.

WO 00/17481 tries to overcome those disadvantages by no longer making use of distinctive straight rod sections, but by making use of a semi-rigid elongate probing rod that is wound in one piece onto a large diameter storage wheel. During penetration the elongate probing rod then can be gradually unwound from the wheel and forced to penetrate into the ground by suitable drive means, that here are formed by endless caterpillar-like engaging elements.

A disadvantage with this however is that the probing rod during its unwinding of the large diameter storage wheel needs to be plastically deformed such that it gets straight. When the penetration process is completed, and the probing rod needs to be pulled out of the ground, the probing rod again needs to be plastically deformed such that it can be wound back again on the large diameter storage wheel. Those repeated plastic deformations make it necessary to have the probing rod replaced after a limited number of usages. This makes this type of soil probing device costly. Another disadvantage is that the required plastic deformation makes it necessary to use a relative slender elongate probing rod, because otherwise the plastic deformation would become too difficult. In particular, with this type of elongate probing rod in one piece, a diameter of 19 mm is known to be used, whereas for probing rods composed out of distinctive rod sections, on almost twice as large diameter of 36 mm is more common. However such a relative slender probing rod has limited strength and limits the maximum penetration depth. Also it entails the risk that it may start to plastically deform and deviate from its aimed vertical penetration path when running against obstacles.

The present invention aims to overcome those disadvantages at least partly and/or to provide a usable alternative. In particular the present invention aims to provide a user-friendly and cost-efficient soil probing device that can be used even at great depths under sea level, where it is hardly possible for operators or maintenance personnel to perform actions to the device like (dis)connecting blocked probing rod sections or trying to solve other types of malfunctions.

This aim is achieved by a soil probing device according to claim 1. The device comprises a measuring probe and a plurality of rod sections for the assembly of a probing rod. Driving means are provided for penetrating the probing rod into the ground. During its penetrating into the ground, the probing rod is extendable each time by a new one of the rod sections. Measuring means are provided for determining properties of the ground during this penetration. The rod sections are provided at their outer ends with complementary male and female locking parts. In an axially aligned position of adjacent rod sections, those locking parts are movable relative to each other from an unlocked into a locked position and vice versa. According to the inventive thought the male locking part comprises two or more threaded male bayonet columns. Each threaded male bayonet column comprises a plurality of radially outwardly projecting screw thread turn sections that lie above one another in an axial direction. The threaded male bayonet columns are spaced apart in a circumferential direction with slit-shaped spacings lying between them. The female locking part comprises two or more threaded female bayonet columns. Each threaded female bayonet column comprises a plurality of radially inwardly projecting screw thread turn sections that lie above one another in the axial direction. The threaded female bayonet columns are spaced apart in the circumferential direction with slit-shaped spacings lying between them. A lower locking part of the new one of the rod sections is insertable into an upper locking part of the probing rod towards an inserted position. In this inserted position the threaded male bayonet columns have come to lie in the slit-shaped spacings of the female locking part, and the threaded female bayonet columns have come to lie in the slit-shaped spacings of the male locking part. The plurality of the respective male and female screw thread turn sections subsequently are simultaneously engageable with each other by means of a rotation of the new one of the rod sections around its central axis over a rotation angle of less than 360 degrees relative to the probing rod towards the locked position.

This advantageously makes it possible for the rod sections to be quickly and easily connected to one another. The alternating threaded columns and spacings of the male and female locking parts can easily be slid into each other in the axial direction, after which the plurality of complementary male and female screw thread turn sections can all at the same time be engaged into each other by means of merely a limited rotation of less than a full turn, in particular even less than 90 degrees. Thus the invention combines the strength of a screw thread connection that has been screwed over a plurality of turns into each other, with the ease of a bayonet-type of insert/rotate connection movement. It has appeared that the thus obtained connections between the rod sections are more than strong enough for the aimed penetration of the probing rod into the ground. The connection process of a new rod section to the probing rod can be performed such easy and quickly that it does not have to negatively influence any penetration measurements that are performed at the same time. The connection process requires little space upstream of the drive means, and thus makes the building of a compact device possible.

In a preferred embodiment a pusher can be provided for pushing the new one of the rod sections with its lower locking part towards said inserted position with the upper locking part of the probing rod, and a rotator can be provided for subsequently rotating the new one of the rod sections with its lower locking part towards said locked position with the upper locking part of the probing rod. Thus the connection process of a new rod section to the probing rod can easily be automated, and make the probing device suitable for performing penetration tests at locations where operators and maintenance personnel have no access, for example at deep sea levels under water. For example a robot arm can be provided that is designed to each time grip a new rod section out of a storage magazine, and then have its locking part inserted into the one of the probing rod and then rotate it into its locked position. Such a robot arm can be built to fit inside a relative compact frame including the storage magazine for the plurality of rod sections.

In another preferred embodiment the threaded male and female bayonet columns at their axially outer head sides can be provided with complementary sliding faces. The sliding faces are angled relative to the circumferential direction. During insertion of the locking part of the new rod section in the axial direction into the upper locking part of the probing rod, the sliding faces shall automatically first come to lie wholly or partly against each other and shall cause the new one of the rod sections to rotate around its central axis until its lower locking part reaches a rotationally aligned position with the locking part of the probing rod. In this rotationally aligned position the threaded male bayonet columns have come to lie in front of the slit-shaped spacings of the female locking part, while the threaded female bayonet columns have come to lie in front of the slit-shaped spacings of the male locking part. A further exerting of a downwards directed pushing force onto the new rod section then shall cause it to easily slide straight along the axial direction from this rotationally aligned and partly inserted position towards the fully inserted position into the locking part of the probing rod. When in this fully inserted position, the new one of the rod sections can easily and quickly be rotated towards the securely locked position.

In a further embodiment the pusher can be designed to exert a downwards directed pushing force to the new one of the rod sections without this pushing force standing in the way of the new one of the rod sections at a same time starting to rotate around its central axis towards its rotationally aligned position because of the complementary wedge shapes of the cooperating sliding faces. This has the advantage that the drive means shall be charged with less momentum forces during the insertion process.

In a preferred variant each angled sliding face may be delimited towards its adjacent slit-shaped spacings by stop edges that extend parallel to the central axis of its rod section. These stop edges then can prevent that the new rod section gets rotated past by the rotationally aligned position because of front side stop edges of one of the locking parts bumping against backside stop edges of the other locking part and/or because of leading ends of the screw thread turn sections bumping against those backside stop edges. This efficiently prevents that merely outermost ones of the screw thread turn sections of the respective bayonet columns may start to prematurely already start to engage into each other before the columns have been able to take in their fully inserted position.

In yet another embodiment the male and female locking parts may be equipped with limitation edges that abut against each other in the inserted position. The screw thread turn sections of the threaded male and female bayonet columns then can be dimensioned to fit with a play in the axial direction between each other during a first part of a rotation of the new one of the rod sections relative to the probing rod from its fully inserted position towards the locked position. With a play in the axial direction it is meant that widths between the respective screw thread turn sections are dimensioned larger than the screw thread sections themselves. Owing to this, the screw thread turn sections of the lower locking part of the new one of the rod sections, during the first part of the rotation, have enough free space in between the screw thread turn sections of the probing rod such that they can be rotated into each other without immediately starting to exert pulling forces onto each other in the axial direction. This has the advantage that the first part of the rotation requires very little rotation force of the rotator. Also it entails the advantage that the locking parts in their fully inserted position already may come to lie against some kind of rigid limitation faces of each other. It is not necessary to provide an axially compressible element between them, like a sealing ring, neither is it necessary to carefully axially position them relative to each other. Owing to the axial play, in this fully inserted unlocked position, free outer ends of the screw thread turn sections do not bump against each other and thus are unable to block the rotation towards the locked position. Depending on the amount of initial axial play and depending on the pitch angle of the screw thread turn sections, a certain amount of rotation of the locking parts from the inserted position towards the locked position is able to take away this play. This certain amount of rotation then corresponds to the first part of the rotation for which hardly any momentum is required from the rotator. A further rotating of the locking parts over a second part of the rotation towards the locked position then shall start to exert pulling forces onto each other which firmly tightens them together in the axial direction. Depending on the desired engagement force, this shall require more or less momentum from the rotator.

The above-mentioned limitation faces may for example be formed by the male locking part comprising an axially inner limitation edge against which an axially outer limitation edge of the female locking part abuts in the inserted position.

In a further embodiment the axial play preferably may lie between 0.1-0.8 times a pitch of the screw thread turn sections. This has the advantage that the first part of the rotation extends over a first angle that is sufficiently large for the screw thread turn sections to engage far enough into each other that such that they are well able to take up axial forces as soon as the axial tightening during the second part of the rotation starts. Also it has the advantage that the second part of the rotation extends over a second angle that is sufficiently large to be able to reach the aimed engagement force. In a more particular embodiment the initial axial play shall be about one fifth of the pitch of the screw thread turn sections. For a probing rod with a diameter of between 20-40 mm, and a pitch angle of the screw thread turn sections of between 1-5 degrees, this shall be a play of between 0.1-1.0 mm.

In a variant the male and female locking parts may comprise end portions that fit with a radial play inside each other. For example the male coupling part may comprise a reduced end edge that has radial dimensions that are smaller than the radial dimensions of its slit-shaped spacings. The reduced end edge then can serve as temporary support for an inner wall part of a female locking part, with this female locking part having its central axis angled relative to the central axis of the male locking part. This in turn makes it possible for a new rod section to get manoeuvred towards the probing rod not only when already fully aligned therewith but also starting from a sideways slanted position.

In a variant at least three of the threaded male bayonet columns and at least three of the threaded female bayonet columns are provided that are equally distributed over the circumference of their respective male and female locking parts. Thus a symmetrical engagement of the columns into each other is possible, which helps to equally distribute the pulling forces over the locking parts and also helps to keep the rotating towards the axially aligned position and/or towards the locked position limited to less than 60 degrees.

Each threaded bayonet column preferably may comprise at least five screw thread turn sections lying above one another in the axial direction. This has appeared to provide sufficient strength to the probing rod in the locked position.

Further advantageous embodiments are stated in the dependent subclaims.

The invention also relates to a method for determining properties of soil during penetration of a probing rod into the ground using a soil probing device according to one of the preceding claims.

The invention shall be explained in further detail below with reference to the accompanying drawings, in which:
- Fig. 1a-b show front and perspective partly cross-sectional views of an embodiment of a rod section with male and female locking parts for a soil probing device according to the invention;
- Fig. 2a-e show subsequent connection steps of locking parts of adjacent ones of the rod sections of fig. 1, with lower parts of fig. 2e-d showing enlarged views of engaging screw thread sections of the locking parts;
- Fig. 3a-b show a string of flexibly connected ones of the rod sections of fig. 1 and 2 in subsequent phases of their connection steps;
- Fig. 4a-b show an alternative flexible connection for the string of fig. 3;
- Fig. 5a-c show schematic perspective drawings of an embodiment of the soil probing device according to the invention;
- Fig. 6a-b are views according to fig. 1b-c with part of a storage wheel of the device drawn with broken lines;
- Fig. 7a-b shows driving means and a sprocket wheel of fig. 4 and 5 during penetration and removal of a probing rod; and
- Fig. 8a-d shows the use of a rotator during subsequent connection steps of a new one of the rod sections with the probing rod of fig. 5 and 6.

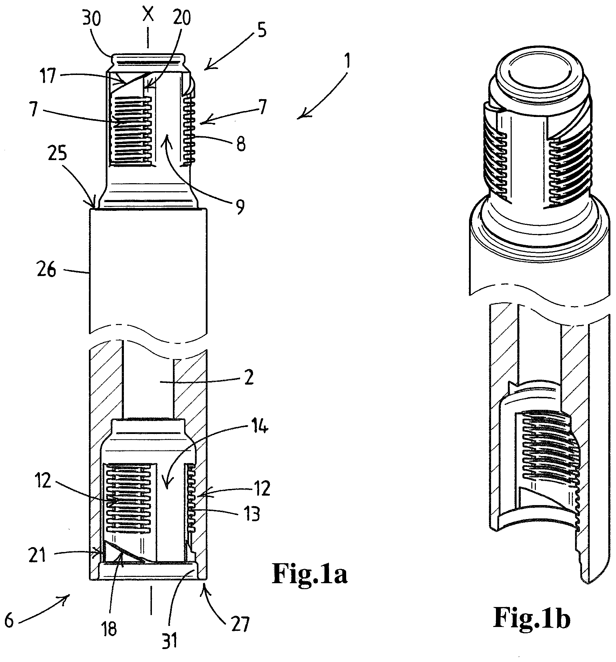

In fig. 1 a rod section for a probing rod is indicated in its entirety with the reference numeral 1. The rod section 1 is formed by a straight hollow tube that delimits a hollow channel 2 with a central axis that extends in an axial direction x. The rod section 1 comprises a male locking part 5 at its upper free end and a complementary female locking part 6 at its lower free end. With this the hollow channel 2 extends through the entire rod including through both the locking parts 5, 6.

The male locking part 5 has a reduced outer diameter relative to the rest of the rod section 1 and comprises three spaced apart threaded male bayonet columns 7 that are equally divided around its circumference. Each column 7 comprises an array of a plurality of right-handed screw thread turn sections 8 that lie above one another in the axial direction x. Slit-shaped smooth spacings 9 lie in between the columns 7.

The female locking part 6 has an increased inner diameter relative to the rest of the channel 2 and comprises three spaced apart threaded female bayonet columns 12 that are equally divided around its circumference. Each column 12 comprises an array of a plurality of right-handed screw thread turn sections 13 that lie above one another in the axial direction x. Slit-shaped smooth spacings 14 lie in between the columns 12.

Each of the screw thread turn sections 8, 13 extends over less than one sixth of the circumference seen in a circumferential direction r. Each of the spacings 9, 14 extends over more than one sixth of the circumference seen in the circumferential direction r.

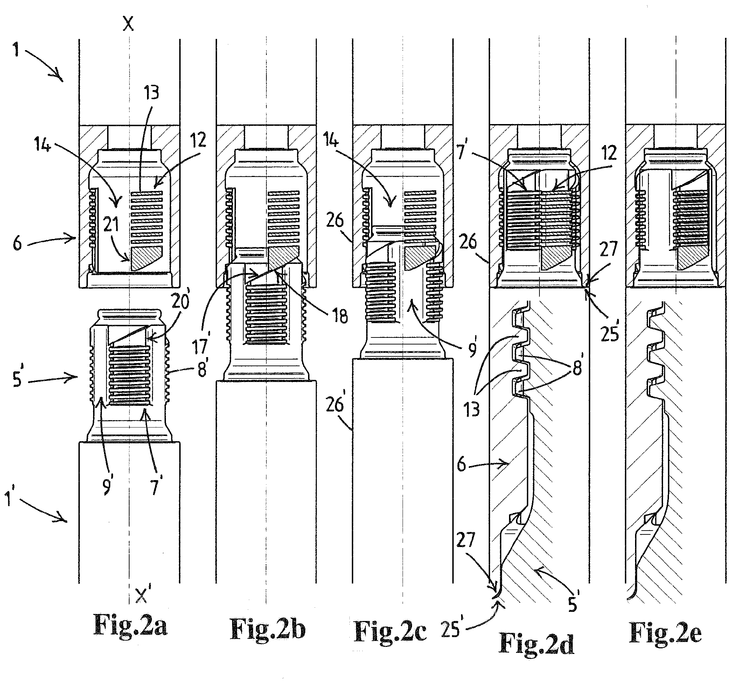

Each of the screw thread turn sections 8, 13 has a pitch angle that lies between 0.5-2.5 degrees. The ridges of the screw thread turn sections 8, 13 are dimensioned smaller than the channels lying between them such that the ridges of one locking part fit with an axial play between the ridges of the other locking part (see also fig. 2d-e). The axial play here is about one fifth of a pitch of the screw thread turn sections 8, 13.

The columns 7 and 12 comprise complementary male and female angled sliding faces 17, 18 at their outer head sides. Like the screw thread turn sections 8, 13, the angled sliding faces 17, 18 also extend over less than one sixth of the circumference and leave the slit-shaped spacings 9, 14 free between them. The angled sliding faces 17, 18 have a pitch angle that is larger than 15 degrees.

Each male angled sliding face 17 towards its front side, here seen in a right-handed locking direction of the circumferential direction r, is delimited by a stop edge 20. The stop edge 20 extends in the axial direction x in the axial prolongation of front side leading ends of the male screw thread turn sections 8.

Each female angled sliding face 18 towards its back side, again seen in the right-handed locking direction, is delimited by a stop edge 21. The stop edge 21 extends in the axial direction x in the axial prolongation of back side tail ends of the female screw thread turn sections 13.

The male locking part 5 at its axial inward end comprises a limitation edge 25 that is formed by a radially extending transition wall part that extends between the male locking part 5 and an outer circumferential wall 26 of the rod section 1. The female locking part 6 at its axial outer end comprises a limitation edge 27 that is formed by a radially extending transition wall part that extends between the female locking part 6 and the outer circumferential wall 26 of the rod section 1.

The male locking part 5 comprises a reduced cylindrical end portion 30 that fits with a radial play inside a widened cylindrical end portion 31 of the female locking part 6.

A connecting of the male and female lock parts 5, 6 of two adjacent ones of the rod sections 1 shall now be explained with reference to fig. 2a-e which show subsequent steps thereof.

In fig. 2a a lower female locking part 6 of an upper rod section 1 and an upper male locking part 5' of a lower rod section 1' are shown spaced apart from each other but already brought in an axially aligned position in which their respective central axes x, x' lie in each other's prolongation.

In fig. 2b the male locking part 5' has been pushed in the axial direction x to start inserting into the female locking part 6 until the angled sliding face 17' has come to lie against the angled sliding face 18.

In fig. 2c it is shown that the male locking part 5' remains being pushed in the axial direction x to insert further into the female locking part 6. With this the sliding faces 17', 18 cause the male locking part 5' to rotate in the right-handed rotational direction r relative to the female locking part 6.

This relative rotation continues until a rotationally aligned position has been reached. In this rotationally aligned position the threaded male bayonet columns 7' have come to lie exactly in line with the slit-shaped spacings 14 of the female locking part 6, while the threaded female bayonet columns 12 have come to exactly lie in line with the slit-shaped spacings 9' of the male locking part 5. It is noted that owing to the provision of the stop edges 20, 21 it is prevented that the male locking part 5' accidentally may get rotated past by the rotationally aligned position, because leading ends of the screw thread turn sections 8', 13 abutting against respective ones of the stop edges 20, 21.

In fig. 2d it is shown that a fully inserted position of the male locking part 5' inside the female locking part 6 has been reached owing to the continued exerting of the pushing force in the axial direction x. In this fully inserted position the limitation edges 25', 27 have come to lie against each other such that a further inserting is not possible. The outer circumferential walls 26, 26' of the adjacent rod sections 1, 1' then form one continuous smooth wall, without any stepped transitions. Furthermore, in this fully inserted position, the threaded male bayonet columns 7' have come to lie in the slit-shaped spacings 14 of the female locking part 6 in between the threaded female bayonet columns 12 of the female locking part 6, whereas those threaded female bayonet columns 12 themselves have come to lie in the slit-shaped spacings 9' in between the threaded male bayonet columns 7' of the male locking part 5'.

It can be seen in the enlarged lower part of fig. 2d that in the shown fully inserted position, the screw thread ridges of the male screw thread sections 8' lie with axial play in front of screw thread channels in between the screw thread ridges of the female screw thread sections 13, and vice versa.

In fig. 2e it is shown that, as a final connection step, the male locking part 5' is rotated in the right-handed rotational direction over an angle of approximately 60 degrees into the locked position. In this locked position each of the respective male and female screw thread turn sections 8', 13 have simultaneously fully engaged into each other.

Owing to the provided axial play, during a first part of this engagement rotation, lower side walls of the screw thread ridges of the male and female screw thread turn sections 8', 13 do not come to lie against each other, but merely start to axially move towards one another until the entire axial play between them is consumed. Then during a second part of the engagement rotation, the lower side walls of the screw thread ridges as well as the abutting limitation edges 25', 27 shall be pulled progressively tighter against each other. This can be seen in the enlarged lower part of fig. 2e.

If it is desired to disconnect the rod sections from each other during a pulling of the probing rod out of the ground again, then the above steps can be performed in the opposite direction, that is to say first performing a rotational left-handed rotation over an angle of approximately 60 degrees until trailing ends of the screw thread turn sections come to abut against respective ones of the stop edges, and then pulling the male and female locking parts out of each other in the axial direction.

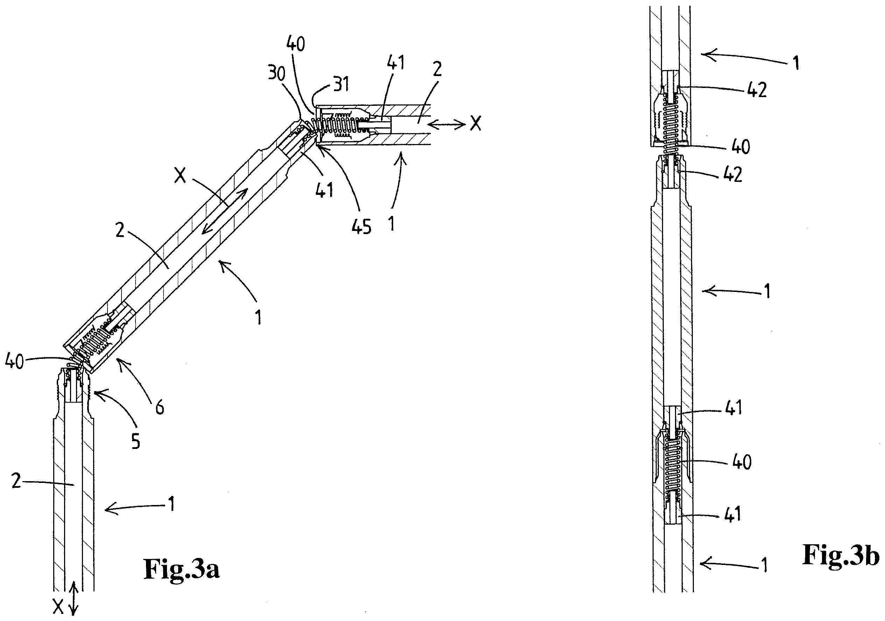

In fig. 3a-b it is shown that adjacent ones of the rod sections 1 together form a flexible string by being connected to each other by means of flexible connection organs that here are formed by coil springs 40. Each coil spring 40 extends through a respective one of the male and female locking parts 5, 6. The outer ends of the coil spring 40 are connected to pistons 41 that are slidably and rotatably guided inside the cylinder-shaped channels 2. The pistons 41 are prevented from being pulled out of the channel 2 by means of inwardly projecting retainer organs 42 that are provided inside the channel 2.

As can be seen in fig. 3a the coil springs 40 can be bend such that in the unlocked non-inserted position of the male and female locking parts 5, 6 of adjacent rod sections 1, those adjacent rod sections 1 are able to take non-aligned sideways bend positions relative to each other. In this non-aligned position the adjacent straight rod sections 1 can be angled relative to each other up to angles of 45 degrees or more. If desired it may even be possible for the rod sections 1 to come to lie parallel to each other.

In the angled position the coil spring 40 may have gotten stretched somewhat such that it exerts a slight bending/pulling force onto its adjacent rod sections 1 which tries to bend/pull the adjacent rod sections 1 towards the axially aligned position as is shown in the upper part of fig. 3b. In this axially aligned position the male and female locking parts 5, 6 can easily and quickly be axially inserted into each other and then rotated relative to each other in order to reach their fully inserted locked position as can be seen in the lower part of fig. 3b. During axial insertion of the locking parts 5, 6, one or both of the pistons 41 can follow this sliding movement by freely sliding inwards into the channels 2. If necessary the pistons 41 the can also perform the described alignment rotation by freely rotating inside the channels 2. During its locking rotation, one or both of the pistons 41 can follow this rotational movement by freely rotating inside the channels 2.

As can be seen in fig. 3a, in the non-aligned positions, the widened cylindrical end portions 31 of the female locking parts 6 partly rest against the reduced cylindrical end portions 30 of the male locking parts 5 of their adjacent rod sections. This gives them ample play to already grip somewhat into each other while being able to bend/kink relative to each other from the non-aligned storage position towards the aligned position and vice versa. Together they form imaginary hinge points 45 for the adjacent rod sections 1. Furthermore, those end portions 30, 31 lying partly against each other in bend/kinked positions of the rod sections 1, make it possible to exert a pushing force onto the string of flexibly connected rod sections 1, which pushing force then can be transferred from one rod section 1 to the other along a line that extends through their respective central axes x.

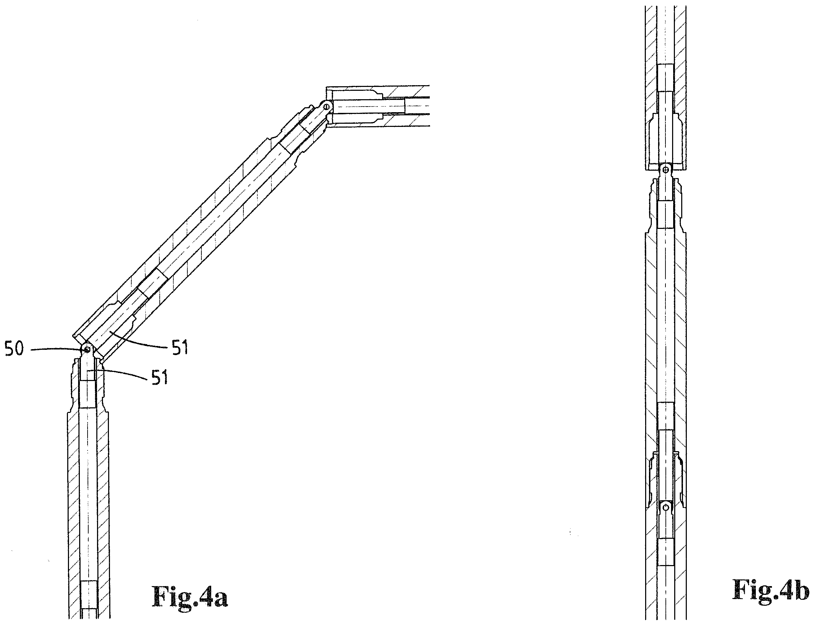

In fig. 4a-b a variant is shown in which the flexible connection organs are formed by a hinge connection 50. The hinge connection 50 has a hinge axis that extends perpendicular to the central axes x of the adjacent rod sections 1. The hinge connection 50 is provided between pistons 51 that extend through the male and female locking parts 5, 6, and that are slidably and rotatably guided inside the cylinder-shaped channels 2. The pistons 51 are prevented from being pulled out of the channel 2 by means of suitable retainer organs that are provided inside the channel 2.

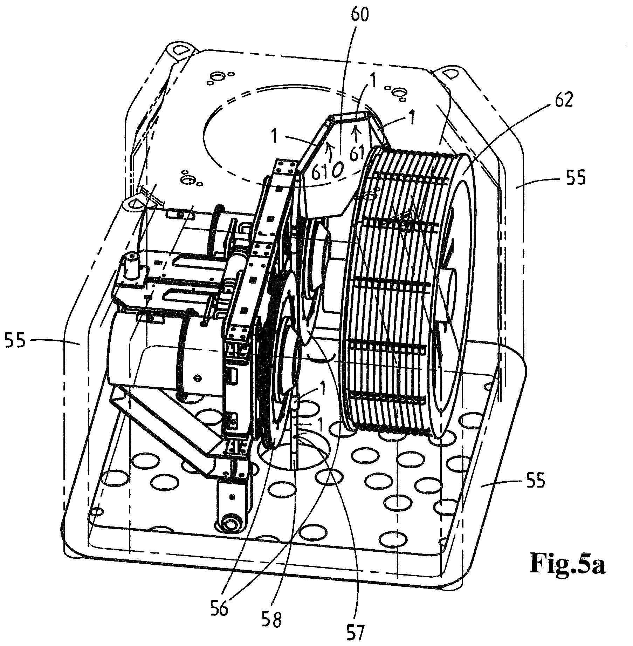

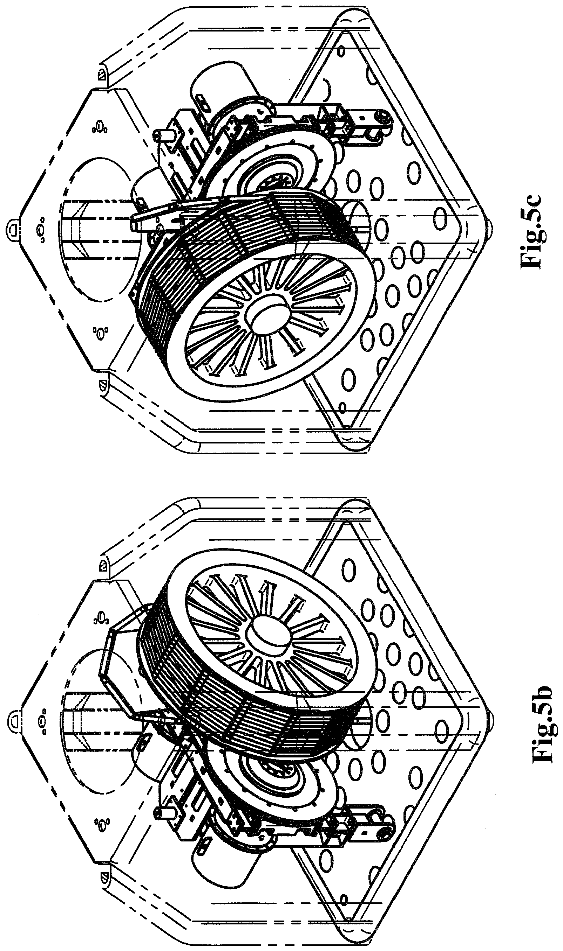

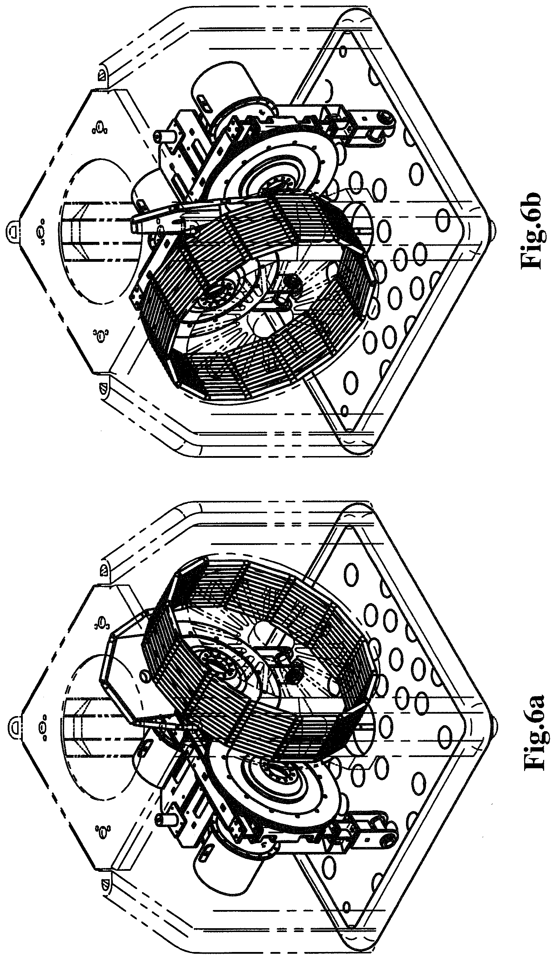

In fig. 5 and 6 an embodiment is shown of an entire soil probing device that makes use of the string of flexibly connected rod sections as shown in fig. 3 or 4 including the male and female locking parts as shown in fig. 1 and 2.

The device is placed inside a frame 55 that can be placed onto a ground of which properties are to be determined, for example a sea floor. The device comprises two grooved rotatable gripping wheels 56 as driving means that are positioned opposite one another. A probing rod 57 having a measuring probe 58 at its lower end and a plurality of rod sections 1 already connected thereto, is guided with such a tight fit in between the gripping wheels 56, that a rotating of the gripping wheels 56 by means of a suitable drive unit, enables them to force the probing rod 57 to penetrate into the ground or to be pulled out of the ground again.

The measuring probe 58 can be connected via a measuring cable that extends through the plurality of rod sections 1 to a control unit (not shown). This control unit is designed for determining properties of the ground during penetration of the probe 58 into the ground. Penetration resistance forces that are active on the probe 58 during this penetration into the ground, and that may vary in dependence of the ground properties, are thus transmitted to the control unit. For example strain gauges can be provided inside or along the measuring probe 58 for measuring those forces. Instead of a measuring cable it is also possible to have the penetration resistance data get transmitted to the control unit wireless, for example via RF.

A sprocket wheel 60 is provided upstream of the wheels 56. The sprocket wheel 60 comprises six flat supporting segments 61. A string of unlocked non-aligned rod sections 1 is guided such over the sprocket wheel 60 that a rotating of the sprocket wheel 60 by means of a suitable drive unit, enables it to repeatedly push a lower locking part of a new one of the rod sections 1 of the string towards the probing rod 57 or to pull it away from it.

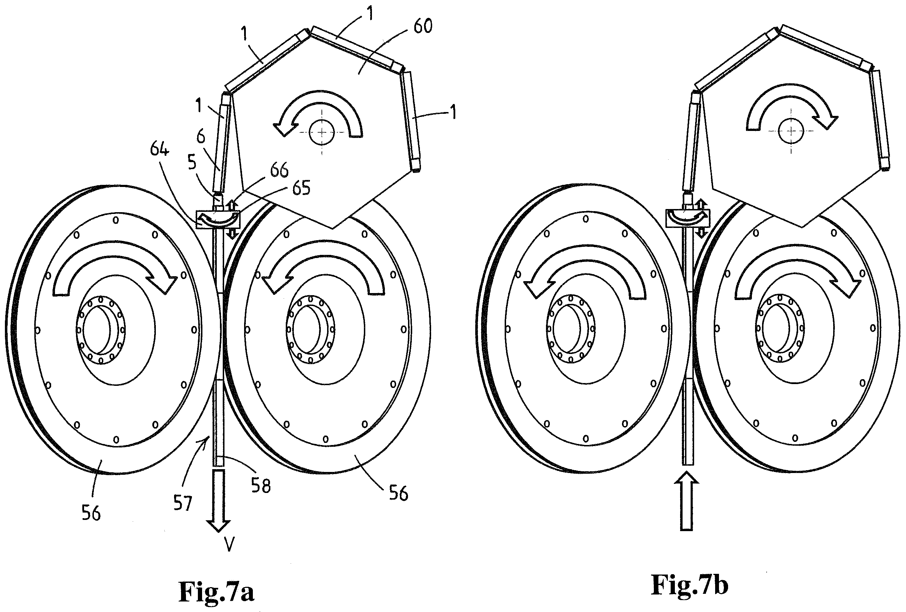

The sprocket wheel 60 is provided at a fixed relative distance from the gripping wheels 56 such that space is given therebetween for an upper part of the last rod section 1 that has already been locked to the probing rod 57 but that still extends above the gripping wheels 56, as well as to a new rod section 1 that extends freely unlocked in between the gripping wheels 56 and the sprocket wheel 60, as well as to part of yet another new rod section 1 of the string while it is still partly supported by the sprocket wheel 60 (see also fig. 7). The freely extending unlocked rod section 1 thus has enough space to gradually bend/kink and rotate from its non-aligned angled position towards its aligned position to then get locked onto the probing rod 57.

A storage wheel 62 is provided upstream of the sprocket wheel 60 at a position sideways of the gripping wheels 56. The storage wheel 62 is formed by a reel having a relative large winding diameter. The string of unlocked angled rod sections 1 can be unwound from or wound upon the storage wheel 62 with distinctive turns of the string getting positioned side by side of each other. A total length of for example more than 50 meter of string can thus be stored onto the storage wheel 62. With this the storage wheel 62 gets rotated by means of a suitable drive unit. The sprocket wheel is supported with its rotation axis such that it is able to function as a level winder. For this it is able to hinge around a vertical axis. The storage wheel 62 is provided with a spiral groove for neatly taking up the respective string turns side by side.

The operation of the device shall now be explained with reference to fig. 7 where merely the gripping wheels 56, the probing rod 57, the string of rod sections 1 and the sprocket wheel 60 of the device are shown.

In fig. 7a it can be seen that the gripping wheels 56 are driven in opposite rotational directions. The driving takes place at a constant speed such that the probing rod 57 gets downwardly pushed at a constant speed v of for example a few centimetres per second. The clamping force that is exerted by the gripping wheels 56 onto the probing rod 57 is such that the chance of vertical slip is small while a chance for the probing rod 57 starting to rotate is neglectable.

In fig. 7a it can also be seen that the sprocket wheel 60 is driven in rotation. This driving however takes place at constant tension, such that the upstream string of rod sections 1 is able to follow the constant speed of the downstream probing rod 57 while at the same time accomplishing the required axial sliding of the lower locking part 6 of a new one of the rod sections 1 over the upper locking part 5 of the probing rod 57. Thus the sprocket wheel 60 functions as a pusher.

The device also comprises a rotator 64 that has merely been schematically indicated in fig. 7. This rotator 64 is designed to give a rotational locking force to the new rod section 1 as soon as it is in the fully inserted position relative to the probing rod 57. The rotator 64 is designed to temporarily clamp the rod section 1 and force the locking rotation upon this rod section 1. This is indicated by the arrow 65. The rotator 64 at the same time performs a temporary downward movement at the same speed the probing rod 57 is moved down by the gripping wheels 56. This is indicated by the arrow 66.

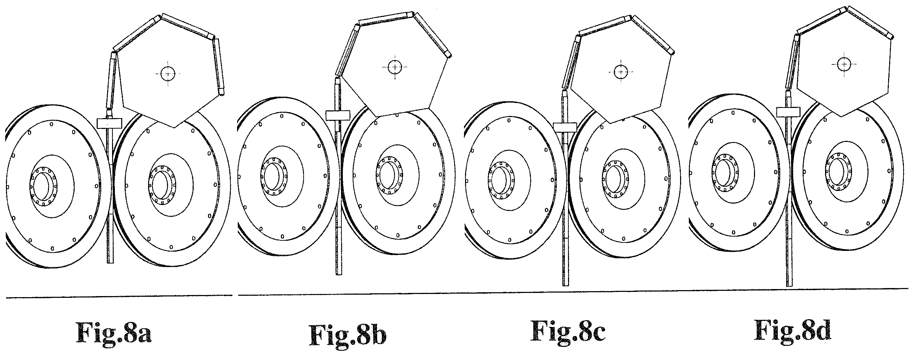

Fig. 8a-d shows the progress of each time connecting a new rod section 1 to the probing rod 57 while it is moved towards and penetrated into the ground. It can be seen in fig. 8b-c that when the rotator 64 screws a rod section 1 to the probing rod 57, the sprocket wheel 60 already starts to feed a new rod section 1. Also the up and down movement of the rotator 64 can be seen there.

During the entire penetration process, the rod sections 1, owing to their flexible connections, are well able to get transported from the storage wheel 62 via the sprocket wheel 60 towards the gripping wheels 56. The male and female locking parts 5, 6 are always in correct positions relative to each other, whereas the control of the various wheels of the device can be mainly passive by means of the driving at continuous speed or tension. Only the rotator 64 needs to be driven actively, for which the control information can come from a detection of the rotational position of the sprocket wheel 60 in combination with status information about the device whether it is resting, penetrating or retracting. With the aid of this position detection of the sprocket wheel 60, a control unit can permanently determine the position of the new rod section 1 that is present in the space between the gripping and sprocket wheels 56, 60, and then at pre-programmed positions activate the rotator 64 for starting to screw that rod section 1 onto the probing rod 57.

The device as shown in fig. 5 and 6 can not only be used for penetrating the probing rod 57 into the ground, it can also be used for pulling the probing rod 57 out of the ground again as soon as the penetration test has been completed. Fig. 7b shows the situation during such a retraction of the probing rod 57 out of the ground again. For this the driving directions of the various wheels and of the rotator are reversed. The gripping wheels 56 then preferably get driven at a higher reversed speed such that the probing rod 57 gets pulled out of the ground as quickly as possible. The rotator 64 then gets controlled to each time unscrew the uppermost rod section 1 of the probing rod 57, whereas the sprocket wheel 60 then pulls this unlocked rod section 1 out of its fully inserted position towards an angled non-aligned position. The string of unlocked rod sections 1 then gets guided by the sprocket wheel 60 towards the storage wheel 6 where it gets coiled around.

Besides the shown embodiments numerous variants are possible. For example the device can be placed permanently on a vehicle for performing penetration test on land instead of in a frame that needs to get positioned onto a piece of ground. It is also possible to use other types of driving means, like two sets of clamping members that can be moved up and down independently from each other and that are designed to alternately clamp a probing rod and push it gradually into the ground. Instead of having the probing rod get penetrated in one continuous go into the ground it is also possible to perform discontinuous penetration tests in which the driving means get stopped each time a new rod section needs to get connected thereto. Instead of a pusher and/or a rotator being provided, it is also possible to connect or disconnect the rod sections manually to or from the measuring probe. Instead of using the string of flexibly connected rod sections it is also possible to use a pick and place unit that each time picks a distinctive separate rod section out of a storage and place this with its locking part onto the probing rod or to perform this manually. Then also the threaded bayonet-type of locking parts are already able to perform an advantageous role.

Thus the present invention advantageously is able to provide a reliable and efficiently operating soil probing device which makes use of threaded bayonet-type of locking parts that can get quickly and automatically connected to a probing rod while this rod is penetrating into the ground.

## Claims

1. Soil probing device comprising:
- a measuring probe (58);
- a plurality of rod sections (1) for the assembly of a probing rod (57) comprising the measuring probe (58), each rod section (1) having a central axis (x);
- driving means (56) for penetrating the probing rod (57) into the ground, which probing rod (57), while being penetrated into the ground, is extendable each time by a new one of the rod sections (1);
- measuring means for determining properties of the ground during penetration of the probing rod (57) into the ground;
in which the rod sections (1) are provided at their outer ends with complementary male and female locking parts (5, 6) that, in an axially aligned position of adjacent rod sections (1), are movable relative to each other from an unlocked into a locked position and vice versa,
**characterized in that,**
the male locking part (5) comprises two or more threaded male bayonet columns (7), each threaded male bayonet column (7) comprising a plurality of radially outwardly projecting screw thread turn sections (8) lying above one another in an axial direction (x), wherein the threaded male bayonet columns (7) are spaced apart in a circumferential direction (r) with slit-shaped spacings allying between them,
the female locking part (6) comprises two or more threaded female bayonet columns (12), each threaded female bayonet column (12) comprising a plurality of radially inwardly projecting screw thread turn sections (13) lying above one another in the axial direction, wherein the threaded female bayonet columns (12) are spaced apart in the circumferential direction with slit-shaped spacings (14) lying between them,
wherein a lower locking part (5, 6) of the new one of the rod sections (1) is insertable with an upper locking part (5, 6) of the probing rod (57) towards an inserted position with the threaded male bayonet columns (7) coming to lie in the slit-shaped spacings (14) of the female locking part (6), and with the threaded female bayonet columns (12) coming to lie in the slit-shaped spacings (9) of the male locking part (5), and
wherein the plurality of the respective male and female screw thread turn sections (8, 13) subsequently are simultaneously engageable with each other by means of a rotation of the new one of the rod sections (1) around its central axis over a rotation angle of less than 360 degrees relative to the probing rod (57) towards said locked position.

2. Soil probing device according to claim 1, wherein a pusher (60) is provided for pushing the new one of the rod sections (1) with its lower locking part (5, 6)towards said inserted position with the upper locking part (5, 6) of the probing rod (57), and wherein a rotator (64) is provided for subsequently rotating the new one of the rod sections (1) with its lower locking part (5, 6)towards said locked position with the upper locking part (5, 6) of the probing rod (57).

3. Soil probing device according to claim 1 or 2, wherein the threaded male and female bayonet columns (7, 12) at their axially outer head sides are provided with complementary sliding faces (17, 18)that are angled relative to the circumferential direction (r) and that together cause the new one of the rod sections (1) to rotate with its lower locking part (5, 6) around its central axis (x) towards a rotationally aligned position during its insertion in the axial direction (x) into the upper locking part (5, 6) of the probing rod (57), in which rotationally aligned position the threaded male bayonet columns (7) have come to lie in front of the slit-shaped spacings (14) of the female locking part (6), while the threaded female bayonet columns (12) have come to lie in front of the slit-shaped spacings (9) of the male locking part (5).

4. Soil probing device according to claims 2 and 3, wherein the pusher (60) is designed to exert a downwards directed pushing force to the new one of the rod sections (1) while this new one of the rod sections (1) at a same time has freedom to rotate around its central axis (x) towards said rotationally aligned position.

5. Soil probing device according to one of the preceding claims, wherein the angled sliding faces (17, 18)are delimited by stop edges (20, 21)towards the slit--shaped spacings, which stop edges (20, 21)extend parallel to the central axis (x) of its rod section (1).

6. Soil probing device according to one of the preceding claims, wherein the male and female locking parts (5, 6)comprise limitation edges (25, 27) that abut against each other in the inserted position, and wherein the screw thread turn sections (8) of the threaded male bayonet columns (7) and the screw thread turn sections (13) of the threaded female bayonet columns (12) are dimensioned to fit with a play in the axial direction (x) between each other during a first part of the rotation around its central axis (x) of the new one of the rod sections (1) relative to the probing rod (57)towards the locked position.

7. Soil probing device according to claim 6, wherein the play lies between 0.1-0.8 times a pitch of the screw thread turn sections (8, 13).

8. Soil probing device according to one of the preceding claims, wherein the male and female locking parts (5, 6)comprise end portions (30, 31) that fit with a radial play inside each other.

9. Soil probing device according to one of the preceding claims, wherein at least three of the threaded male bayonet columns (7) and at least three of the threaded female bayonet columns (12) are provided that are equally distributed over the circumference of their respective male and female locking parts (5, 6).

10. Soil probing device according to one of the preceding claims, wherein each threaded bayonet column (7, 12) comprises at least five screw thread turn sections (8, 13)lying above one another in the axial direction (x).

11. Soil probing device according to one of the preceding claims, wherein each screw thread turn section (8, 13) extends over less than a quarter of the circumference of its locking part (5, 6).

12. Method for determining properties of soil during penetration of a probing rod (57) into the ground using a soil probing device according to one of the preceding claims, comprising the steps of:
- penetrating the probing rod (57) into the ground, while extending it each time by a new one of the rod sections (1);
- determining properties of the soil during penetration of the probing rod (57) into the ground;
**characterized in that,**
a lower locking part (5, 6) of the new one of the rod sections (1) is inserted into the upper locking part (5, 6) of the probing rod (57) towards the inserted position with the threaded male bayonet columns (7) coming to lie in the slit-shaped spacings 14) of the female locking part (6), and with the threaded female bayonet columns (12)coming to lie in the slit-shaped spacings (9) of the male locking part (5), and
wherein the plurality of the respective male and female thread turn sections (8, 13) subsequently are simultaneously engaged with each other by means of a rotation of the new one of the rod sections (1) around its central axis (x) over a rotation angle of less than 360 degrees relative to the probing rod (57) towards the locked position.

## Patentansprüche

1. Bodensondiervorrichtung umfassend:
- eine Messsonde (58);
- eine Vielzahl von Stangenabschnitten (1) für die Montage einer Sondenstange (57) umfassend die Messsonde (58), wobei jeder Stangenabschnitt (1) eine Mittelachse (x) aufweist;
- Antriebsmittel (56) zum Eindringen der Sondenstange (57) in den Boden, wobei die Sondenstange (57), während des Eindringens in den Boden, jedes Mal durch einen neuen der Stangenabschnitte (1) erweiterbar ist;
- Messmittel zum Bestimmen von Eigenschaften des Bodens während des Eindringens der Sondenstange (57) in den Boden; wobei die Stangenabschnitte (1) an ihren äußeren Enden mit komplementären männlichen und weiblichen Verriegelungsteilen (5, 6) versehen sind, die in einer axial ausgerichteten Position benachbarter Stangenabschnitte (1) relativ zueinander von einer entriegelten in eine verriegelte Position und umgekehrt bewegbar sind,
**dadurch gekennzeichnet, dass**
das männliche Verriegelungsteil (5) zwei oder mehr männliche Bajonettsäulen (7) mit Gewinde umfasst, wobei jede männliche Bajonettsäule (7) mit Gewinde eine Vielzahl von radial nach außen vorstehenden Schraubgewinde-Windungsabschnitten (8) aufweist, welche in einer axialen Richtung (x) übereinander liegen, wobei die männlichen Bajonettsäulen (7) mit Gewinde in einer Umfangsrichtung (r) beabstandet sind, wobei schlitzförmige Abstände (9) zwischen ihnen liegen,
wobei das weibliche Verriegelungsteil (6) zwei oder mehr weibliche Bajonettsäulen (12) mit Gewinde aufweist, wobei jede weibliche Bajonettsäule (12) mit Gewinde eine Vielzahl von radial nach innen ragenden Schraubgewinde-Windungsabschnitten (13) aufweist, welche in der axialen Richtung übereinander liegen, wobei die weiblichen Bajonettsäulen (12) mit Gewinde in Umfangsrichtung beabstandet sind, wobei schlitzförmige Abstände (14) zwischen ihnen liegen,
wobei ein unterer Verriegelungsteil (5, 6) des neuen der Stangenabschnitte (1) mit einem oberen Verriegelungsteil (5, 6) der Sondenstange (57) zu einer eingeführten Position einführbar ist, wobei die männlichen Bajonettsäulen (7) mit Gewinde in den schlitzförmigen Abständen (14) des weiblichen Verriegelungsteils (6) zu liegen kommen und die weiblichen Bajonettsäulen (12) mit Gewinde in den schlitzförmigen Abständen (9) des männlichen Verriegelungsteils (5) zu liegen kommen und
wobei die Vielzahl der jeweiligen männlichen und weiblichen Schraubgewinde-Windungsabschnitte (8, 13) anschließend gleichzeitig mittels einer Drehung des neuen der Stangenabschnitte (1) um dessen zentrale Achse über einen Drehwinkel von weniger als 360 Grad relativ zur Sondenstange (57) zur verriegelten Position miteinander in Eingriff gebracht werden können.

2. Bodensondiervorrichtung nach Anspruch 1, wobei ein Schieber (60) vorgesehen ist, um den neuen der Stangenabschnitte (1) mit seinem unteren Verriegelungsteil (5, 6) in Richtung der eingeführten Position mit dem oberen Verriegelungsteil (5, 6) der Sondenstange (57) zu schieben und wobei ein Rotator (64) zum nachfolgenden Drehen des neuen der Stangenabschnitte (1) mit seinem unteren Verriegelungsteil (5, 6) in Richtung auf die verriegelte Position mit dem oberen Verriegelungsteil (5, 6) der Sondierungsstange (57) vorgesehen ist.

3. Bodensondiervorrichtung nach Anspruch 1 oder 2, wobei die männlichen und weiblichen Bajonettsäulen (7, 12) mit Gewinde an ihren axial äußeren Kopfseiten mit komplementären Gleitflächen (17, 18) versehen sind, die relativ zu der Umfangsrichtung (r) abgewinkelt sind und die zusammen den neuen der Stangenabschnitte (1) mit seinem unteren Verriegelungsteil (5, 6) um seine Mittelachse (x) in eine in Drehrichtung ausgerichtete Position während seines Einsetzens in der axialen Richtung (x) in das obere Verriegelungsteil (5, 6) der Sondenstange (57) bringen, in welcher ausgerichteten Position die männlichen Bajonettsockel (7) mit Gewinde vor den schlitzförmigen Abständen (14) des weiblichen Verriegelungsteils (6) zu liegen kommen, während die weiblichen Bajonettsäulen (12) mit Gewinde vor den schlitzförmigen Abständen (9) des männlichen Verriegelungsteils (5) zu liegen kommen.

4. Bodensondiervorrichtung nach den Ansprüchen 2 und 3, wobei der Schieber (60) dazu ausgebildet ist, eine nach unten gerichtete Schubkraft auf den neuen der Stangenabschnitte (1) auszuüben, während dieser neue der Stangenabschnitte (1) gleichzeitig die Freiheit hat, sich um seine Mittelachse (x) zur in Drehrichtung ausgerichteten Position zu drehen.

5. Bodensondiervorrichtung nach einem der vorhergehenden Ansprüche, wobei die abgewinkelten Gleitflächen (17, 18) durch Anschlagkanten (20, 21) zu den schlitzförmigen Abständen hin begrenzt sind, welche Anschlagkanten (20, 21) sich parallel zur Mittelachse (x) ihres Stangenabschnitts (1) erstrecken.

6. Bodensondiervorrichtung nach einem der vorhergehenden Ansprüche, wobei die männlichen und weiblichen Verriegelungsteile (5, 6) Begrenzungskanten (25, 27) umfassen, die in der eingeführten Position aneinander anliegen, und wobei die Schraubgewinde-Windungsabschnitte (8) der männlichen Bajonettsäulen (7) mit Gewinde und die Schraubgewinde-Windungsabschnitte (13) der weiblichen Bajonettsäulen (12) mit Gewinde derart dimensioniert sind, dass sie mit einem Spiel in der axialen Richtung (x) zueinander während eines ersten Teils der Drehung um ihre Mittelachse (x) des neuen der Stangenabschnitte (1) relativ zu der Sondenstange (57) in Richtung der verriegelten Position passen.

7. Bodensondiervorrichtung nach Anspruch 6, wobei das Spiel zwischen dem 0,1 bis 0,8-fachen einer Steigung der Schraubgewinde-Windungsabschnitte (8, 13) liegt.

8. Bodensondiervorrichtung nach einem der vorhergehenden Ansprüche, wobei die männlichen und weiblichen Verriegelungsteile (5, 6) Endabschnitte (30, 31) aufweisen, die mit einem radialen Spiel ineinander passen.

9. Bodensondiervorrichtung nach einem der vorhergehenden Ansprüche, bei der mindestens drei der männlichen Bajonettsäulen (7) mit Gewinde und mindestens drei der weiblichen Bajonettsäulen (12) mit Gewinde vorgesehen sind, die gleichmäßig über den Umfang ihrer jeweiligen männlichen und weiblichen Verriegelungsteile (5, 6) verteilt sind.

10. Bodensondiervorrichtung nach einem der vorhergehenden Ansprüche, bei jede der Bajonettsäulen (7, 12) mit Gewinde wenigstens fünf in der axialen Richtung (x) übereinander liegende Schraubgewinde-Windungsabschnitte (8, 13) aufweist.

11. Bodensondiervorrichtung nach einem der vorhergehenden Ansprüche, wobei sich jeder Schraubgewinde-Windungsabschnitt (8, 13) über weniger als ein Viertel des Umfangs seines Verriegelungsteils (5, 6) erstreckt.

12. Verfahren zum Bestimmen von Bodeneigenschaften während des Eindringens einer Sondenstange (57) in den Boden unter Verwendung einer Bodensondiervorrichtung nach einem der vorhergehenden Ansprüche, umfassend die Schritte:
- Eindringen der Sondenstange (57) in den Boden, während sie jedes Mal um einen neuen der Stangenabschnitte (1) erweitert wird;
- Bestimmen von Eigenschaften des Bodens während des Eindringens der Sondenstange (57) in den Boden,
**dadurch gekennzeichnet, dass**
ein unterer Verriegelungsteil (5, 6) des neuen der Stangenabschnitte (1) in das obere Verriegelungsteil (5, 6) der Sondenstange (57) in Richtung der eingeführten Position eingeführt wird, wobei die männlichen Bajonettsäulen (7) mit Gewinde in den schlitzförmigen Abständen (14) des weiblichen Verriegelungsteils (6) zu liegen kommen und wobei die weiblichen Bajonettsäulen (12) mit Gewinde in den schlitzförmigen Abständen (9) des männlichen Verriegelungsteils (5) zu liegen kommen, und
wobei die Vielzahl der jeweiligen männlichen und weiblichen Schraubgewinde-Windungsabschnitte (8, 13) anschließend gleichzeitig durch eine Drehung des neuen der Stangenabschnitte (1) um seine Mittelachse (x) über einen Drehwinkel von weniger als 360 Grad relativ zu dem Sondenstange (57) in Richtung auf die verriegelte Position miteinander in Eingriff gebracht werden.

## Revendications

1. Dispositif de sondage du sol comprenant :
une sonde de mesure (58) ;
une pluralité de sections de tige (1) pour l'assemblage d'une tige de sondage (57) comprenant la sonde de mesure (58), chaque section de tige (1) ayant un axe central (x) ;
un moyen d'entraînement (56) pour faire pénétrer la tige de sondage (57) dans le sol, laquelle tige de sondage (57), tout en pénétrant dans le sol, est extensible chaque fois par une nouvelle section des sections de tige (1) ;
un moyen de mesure pour déterminer des propriétés du sol pendant la pénétration de la tige de sondage (57) dans le sol ;
dans lequel les sections de tige (1) sont prévues, au niveau de leurs extrémités externe, avec des parties de verrouillage mâles et femelles complémentaires (5, 6) qui, dans une position axialement alignée des sections de tige (1) adjacentes, sont mobiles les unes par rapport aux autres d'une position déverrouillée à une position verrouillée et vice versa,
**caractérisé en ce que** :
la partie de verrouillage mâle (5) comprend deux colonnes filetées à baïonnette mâles (7) ou plus, chaque colonne filetée à baïonnette mâle (7) comprenant une pluralité de sections de tour de filetage en saillie radialement vers l'extérieur (8) les unes au-dessus des autres dans une direction axiale (x), dans lesquelles les colonnes filetées à baïonnette mâles (7) sont espacées dans une direction circonférentielle (R) avec des espacements en forme de fente (9) entre elles,
une partie de verrouillage femelle (6) comprend deux colonnes filetées à baïonnette femelles (12) ou plus, chaque colonne filetée à baïonnette femelle (12) comprenant une pluralité de sections de tour de filetage en saillie radialement vers l'intérieur (13) les unes au-dessus des autres dans la direction axiale, dans lequel les colonnes filetées à baïonnette femelles (12) sont espacées dans la direction circonférentielle avec des espacements en forme de fente (14) entre elles,
dans lequel une partie de verrouillage inférieure (5, 6) de la nouvelle section des sections de tige (1) peut être insérée avec une partie de verrouillage supérieure (5, 6) de la tige de sondage (57) vers une position insérée avec les colonnes filetées à baïonnette mâles (7) qui viennent dans les espacements en forme de fente (14) de la partie de verrouillage femelle (6), et avec les colonnes filetées à baïonnette femelles (12) qui viennent dans les espacements en forme de fente (9) de la partie de verrouillage mâle (5), et
dans lequel la pluralité de sections de tour de filetage mâles et femelles (8, 13) respectives peuvent simultanément se mettre en prise entre elles au moyen d'une rotation de la nouvelle section des sections de tige (1) autour de son axe central sur un angle de rotation inférieur à 360 degrés par rapport à la tige de sondage (57) vers ladite position verrouillée.

2. Dispositif de sondage du sol selon la revendication 1, dans lequel un poussoir (60) est prévu pour pousser la nouvelle section des sections de tige (1) avec sa partie de verrouillage inférieure (5, 6) vers ladite position insérée avec la partie de verrouillage supérieure (5, 6) de la tige de sondage (57), et dans lequel un rotateur (64) est prévu pour la rotation consécutive de la nouvelle section des sections de tige (1) avec sa partie de verrouillage inférieure (5, 6) vers ladite position verrouillée avec la partie de verrouillage supérieure (5, 6) de la tige de sondage (57).

3. Dispositif de sondage du sol selon la revendication 1 ou 2, dans lequel les colonnes filetées à baïonnette mâles ou femelles (7, 12) au niveau de leurs côtés de tête axialement externe sont prévues avec des faces coulissantes complémentaires (17, 18) qui sont alignées par rapport à la direction circonférentielle (r) et qui amènent ensemble la nouvelle section des sections de tige (1) à tourner avec sa partie de verrouillage inférieure (5, 6) autour de son axe central (x) vers une position alignée en rotation pendant son insertion dans la direction axiale (x) dans la partie de verrouillage supérieure (5, 6) de la tige de sondage (57), dans laquelle position alignée en rotation, les colonnes filetées à baïonnette mâles (7) viennent en face des espacements en forme de fente (14) de la partie de verrouillage femelle (6), alors que les colonnes filetées à baïonnette femelles (12) viennent en face des espacements en forme de fente (9) de la partie de verrouillage mâle (5).

4. Dispositif de sondage du sol selon les revendications 2 et 3, dans lequel le poussoir (60) est conçu pour exercer une force de poussée dirigée vers le bas sur la nouvelle section des sections de tige (1), alors que cette nouvelle section des sections de tige (1) a en même temps la liberté de tourner autour de son axe central (x) vers ladite position alignée en rotation.

5. Dispositif de sondage du sol selon l'une des revendications précédentes, dans lequel les faces coulissantes coudées (17, 18) sont délimitées par des bords d'arrêt (20, 21) vers les espacements en forme de fente, lesquels bords d'arrêt (20, 21) s'étendent parallèlement à l'axe central (x) de sa section de tige (1).

6. Dispositif de sondage du sol selon l'une des revendications précédentes, dans lequel les parties de verrouillage mâles et femelles (5, 6) comprennent des bords de limitation (25, 27) qui viennent en butée les uns contre les autres dans la position insérée, et dans lequel les sections de tour de filetage (8) des colonnes filetées à baïonnette mâles (7) et les sections de tour de filetage (13) des colonnes filetées à baïonnette femelles (12) sont dimensionnées pour s'adapter avec un jeu dans la direction axiale (x) entre elles pendant une première partie de la rotation autour de l'axe central (x) de la nouvelle section des sections de tige (1) par rapport à la tige de sondage (57) vers la position verrouillée.

7. Dispositif de sondage du sol selon la revendication 6, dans lequel le jeu est compris entre 0,1 et 0,8 fois un pas des sections de tour de filetage (8, 13).

8. Dispositif de sondage du sol selon l'une des revendications précédentes, dans lequel les parties de verrouillage mâles et femelles (5, 6) comprennent des parties d'extrémité (30, 31) qui s'adaptent avec un jeu radial les unes à l'intérieur des autres.

9. Dispositif de sondage du sol selon l'une des revendications précédentes, dans lequel on prévoit au moins trois des colonnes filetées à baïonnette mâles (7) et au moins trois des colonnes filetées à baïonnette femelles (12) qui sont réparties à égale distance sur la circonférence de leurs parties de verrouillage mâles et femelles (5, 6) respectives.

10. Dispositif de sondage du sol selon l'une des revendications précédentes, dans lequel chaque colonne filetée à baïonnette (7, 12) comprend au moins cinq sections de tour de filetage (8, 13) les unes au-dessus des autres dans la direction axiale (x).

11. Dispositif de sondage du sol selon l'une des revendications précédentes, dans lequel chaque section de tour de filetage (8, 13) s'étend sur moins d'un quart de la circonférence de sa partie de verrouillage (5, 6).

12. Procédé pour déterminer des propriétés de sol pendant la pénétration d'une tige de sondage (57) dans le sol en utilisant un dispositif de sondage du sol selon l'une des revendications précédentes, comprenant les étapes consistant à :
faire pénétrer la tige de sondage (57) dans le sol, tout en la prolongeant chaque fois avec une nouvelle section des sections de tige (1) ;
déterminer les propriétés du sol pendant la pénétration de la tige de sondage (57) dans le sol ;
**caractérisé en ce que** :
une partie de verrouillage inférieure (5, 6) de la nouvelle section des sections de tige (1) est insérée dans la partie de verrouillage supérieure (5, 6) de la tige de sondage (57) vers la position insérée avec les colonnes filetées à baïonnette mâles (7) qui se trouvent dans les espacements en forme de fente (14) de la partie de verrouillage femelle (6) et avec les colonnes filetées à baïonnette femelles (12) qui se trouvent dans les espacements en forme de tige (9) de la partie de verrouillage mâle (5), et
dans lequel la pluralité de sections de tour de filetage mâles et femelles (8, 13) respectives sont ensuite simultanément mises en prise entre elles par des moyens de rotation de la nouvelle section des sections de tige (1) autour de son axe central (x) sur un angle de rotation inférieur à 360 degrés par rapport à la tige de sondage (57) vers la position verrouillée.
